# EUROPÄISCHE PATENTSCHRIFT

(11) **EP 0 784 992 B1**
(45) Veröffentlichungstag und Bekanntmachung des Hinweises auf die Patenterteilung: **09.04.2003**
(21) Anmeldenummer: 97100159.9
(22) Anmeldetag: 08.01.1997
(51) Int. Cl.: A61M 37/00, A61N 1/30, A61B 5/00

(54) **Vorrichtung zum Behandeln von malignen Gewebsveränderungen**
Treatment device for malignant changes of tissue
Dispositif de traitement des alterations tissulaires malignes

(30) Priorität: 17.01.1996 DE 19601487
(43) Veröffentlichungstag der Anmeldung: 23.07.1997
(73) Patentinhaber: Micronas GmbH, 79108 Freiburg (DE)
(72) Erfinder: Sieben, Ulrich, Dr.-Phys., 79276 Reute (DE); Wolf, Bernhard, Prof.-Dr., 79252 Stegen (DE); Kraus, Michael, Dr., 79822 Titisee-Neustadt (DE)
(74) Vertreter: Patentanwälte Westphal, Mussgnug & Partner

(56) Entgegenhaltungen:
- EP-A- 0 236 285
- WO-A-94/05361
- WO-A-94/08655
- US-A- 4 003 379
- US-A- 4 402 694

## Beschreibung

Die Erfindung bezieht sich auf eine Vorrichtung zum Behandeln von lebendem Zellgewebe, wobei die Vorrichtung einen Sensor-Aktuatorkopf mit einem pH-Sensor und einer Wirkstoffabgabeeinrichtung mit einem einen Wirkstoff bevorratenden Behälter aufweist, und wobei der pH-Sensor und die Wirkstoffabgabeeinrichtung zur Wirkstoffdosierung in Abhängigkeit der pH-Wertmessung an eine Steuereinrichtung angeschlossenen sind.

Aus der WO-A-9408655, die zur Bildung des Oberbegriffs des Anspruchs 1 herangezogen werden ist, ist eine transdermal wirkende Verabreichungsvorrichtung für Medikamente in dosierter Form mit einem Sensor-Aktuatorkopf bekannt. Damit wird der Haut Flüssigkeit entzogen, diese Flüssigkeit analysiert und das Analyseergebnis zur Steuerung der Wirkstoffzufuhr herangezogen.
Bei der transdermalen Verabreichung des Wirkstoffes wird dieser über den Blutkreislauf zu einem Behandlungsbereich transportiert. Neben einer verzögerten Wirkungsrückmeldung gelangt dabei der Wirkstoff über den Blutkreislauf jedoch auch an Stellen des Körpers, wo er unerwünschte Nebenwirkungen hervorrufen kann.

Bei der Krebstherapie ist es bereits bekannt, Chemotherapeutika einzusetzen, die die pathogenen Organteile, möglichst aber nicht den übrigen Organismus schädigen sollen.
Die systemische und regionale Dosierung der Chemotherapeutika ist jedoch problematisch, da einerseits durch eine entsprechende Wirkstoffkonzentration eine hohe Wirksamkeit gegenüber zum Beispiel einem Tumor angestrebt wird, andererseits jedoch durch unspezifische Aufnahme aber dann die Gefahr der Schädigung des gesunden Gewebes besteht.

Das US-Patent 4 003 379 beschreibt ein sogenanntes "Drug Delivery System", mit dem Medikamente innerhalb des Körpers eines Patienten verabreicht werden können. Die Vorrichtung ist dazu in den Körper implantierbar.
Zur Krebsbehandlung können mit dieser Vorrichtung radioaktive Wirkstoffe überwacht und in Abhängigkeit der Konzentration der Radioaktivität Wirkstoff dosiert verabreicht werden.
Auch hierbei kann aus der Konzentration der Radioaktivität nicht auf die Wirkung des Medikamentes auf den behandelten Gewebebereich rückgeschlossen werden.

Aufgabe der vorliegenden Erfindung ist es, eine Vorrichtung zu schaffen, mit der eine targetorientierte Chemotherapie möglich ist, bei der die Belastung und Schädigung der nicht betroffenen Bereiche des Körpers zumindest weitgehend reduziert ist.

Zur Lösung dieser Aufgabe wird vorgeschlagen, daß der Sensor-Aktuatorkopf zumindest mit dem pH-Sensor und der Wirkstoffabgabeeinrichtung derart ausgebildet ist, daß er innerhalb des Körpers im tumorösen Gewebebereich einsetzbar ist und diesen Behandlungsbereich in Applikationsstellung mit einer Auflage- und Kontaktfläche direkt kontaktiert, daß der pH-Sensor zur Bestimmung der Ansäuerung der unmittelbaren Umgebung dieses tumorösen Gewebebereichs angeordnet ist, und daß der Wirkstoff ein solcher zur Anhebung des pH-Wertes der unmittelbaren Umgebung des tumorösen Gewebebereichs und/oder zur Reduzierung der Ansäuerung der Tumorzellen selbst ist.

Zur Überwachung des Behandlungsbereiches dient der pH-Sensor, durch den die Ansäuerung im Behandlungsbereich bestimmt werden kann.

Änderungen des pH-Wertes lassen Rückschlüsse auf die metabolischen Aktivitäten der Tumor-Zellen zu, so daß dadurch entsprechende Behandlungsanpassungen vorgenommen werden können. Dem liegt die Erkenntnis zugrunde, daß Wachstum und Ausbreitung von Tumoren als ein Prozeß zellulärer Selbst-Organisation betrachtet werden müssen, der, abgesehen von Veränderungen im zellulären Signal-Verarbeitungs-Apparat, wesentlich von der Mikro-Umgebung des Tumors gesteuert wird. Dabei spielt der pH-Wert der Mikroumgebung des Tumors eine zentrale Schlüsselrolle.
Wird beispielsweise ein pH-Sollwert von 7,4 vorgegeben, so erfolgt durch die Steuereinrichtung aufgrund der Messung des vorhandenen pH-Wertes als Istwert, eine geregelte Dosierung des medizinischen Wirkstoffes, bis der Sollwert, im Beispiel pH 7,4, erreicht ist. Der medizinische Wirkstoff kann ein Wirkstoff zur Neutralisation des pH-Gradienten sein. Weiterhin kommt ein Wirkstoff (Antagonist) zur Blockade der Protonenpumpe an den Zellmembranen der Tumorzellen oder ein Wirkstoff zur Blockade der molekularbiologischen Agentien (beispielsweise Antisens-Produkte) in Betracht.
Zur chemischen Beeinflussung wird der medizinische Wirkstoff unmittelbar bei einem zu behandelnden Tumor appliziert und gleichzeitig erfolgt dort auch in unmittelbarer Umgebung des Behandlungsortes mit Hilfe des pH-Sensors und gegebenenfalls weiterer Sensoren während der Behandlung eine laufende Überwachung. Aufgrund der Meßwerte wird die Dosierung entsprechend den Sollwertvorgaben von der Steuereinrichtung angepaßt werden. Es ist somit ein selbständig arbeitender Regelkreis gebildet, durch den eine laufende Dosierungsnachführung mit dem zu applizierten Wirkstoff vorgenommen werden kann.

In Kombination mit der chemischen Beeinflussung des tumorösen Gewebes, können nach einer Ausgestaltung der Erfindung am Sensor-Aktuatorkopf Elektroden zur physikalischen Beeinflussung des tumorösen Gewebebereiches durch elektrische und/oder elektromagnetische Felder mittels Iontophorese zur Anhebung des gemessenen pH-Wertes in der unmittelbaren Umgebung eines tumorösen Gewebebereichs vorgesehen sein.
An die Elektroden kann eine Gleichspannung oder eine Wechselspannung angelegt werden. Auch hierbei wird die Änderung des Feldes in Abhängigkeit von dem jeweiligen pH-Meßwert vorgenommen, so daß auch diesbezüglich ein Regelkreis und somit eine gezielte Behandlung mit "Feedback"vorhanden ist.

Der pH-Sensor kann auf Halbleiterbasis auf der Basis einer Leitfähigkeits- und Impedanzmessung ausgebildet sein, wobei bei einem pH-Sensor auf Halbleiterbasis für diesen vorzugsweise wenigstens ein ionenselektiver Feldeffekttransistor (IS-FET) vorgesehen ist.
Mit einem pH-Sensor auf Halbleiterbasis ist eine hohe Meßgenauigkeit erzielbar und ein Sensor auf der Basis einer Leitfähigkeits- und Impedanzmessung läßt sich in bestimmten Anwendungen (Leber, Magen) einfacher anwenden.

Gegebenenfalls ist zusätzlich zu wenigstens einem pH-Sensor wenigstens ein weiterer Sensor, insbesondere ein Ionen- oder Molekularsensor vorgesehen.
Mit diesen zusätzlichen Sensoren lassen sich außer pH-Wertänderungen auch noch zusätzliche, therapierelevante Änderungen in der Mikroumgebung eines Tumors erfassen und aus diesen zusätzlichen Meßdaten können entsprechende Maßnahmen beim Applizieren des medizinischen Wirkstoffes abgeleitet werden.

Zweckmäßigerweise weist die Wirkstoffabgabeeinrichtung vorzugsweise wenigstens eine poröse Membran und eine Wirkstoff-Zuführung zu dieser Membrane auf, wobei sich bei der Wirkstoff-Zuführung eine Dosiereinrichtung befindet, die an eine Dosiersteuerung angeschlossen ist.
Damit ist eine regional begrenzte und dosierte Zuführung von Wirkstoff möglich.
Dabei bilden der oder die Sensoren sowie die gegebenenfalls vorgesehene, poröse Membrane die Auflage- und Kontaktfläche für den zu behandelnden Gewebebereich.
Bei dieser Auflage- und Kontaktfläche für den zu behandelnden Gewebebereich können auch wenigstens zwei Elektroden zu iontophoretischen Zwecken vorgesehen sind, die über elektrische Leitungen mit einer Spannungsquelle verbunden sind.

Eine Ausführungsform der Erfindung sieht vor, daß der Sensor-Aktuatorkopf eine komplette Funktionseinheit bildet und daß diese insbesondere wenigstens einen Wirkstoff-Vorratsbehälter, eine oder mehrere, mit der porösen Membrane oder dergleichen verbundene Dosiereinrichtung(en) mit Dosiersteuerung sowie zumindest einen pH-Sensor aufweist.
Zusätzlich besteht für eine physikalische Beeinflussung auch die Möglichkeit, daß die Funktionseinheit wenigstens einen pH-Sensor, wenigstens zwei Elektroden zu iontophoretischen Zwecken, eine Spannungsquelle sowie eine Steuereinrichtung aufweist.
Eine erfindungsgemäße Vorrichtung wird als komplette, funktionstüchtige Einheit innerhalb des Körpers eingesetzt und kann dort über einen vorgesehenen Behandlungszeitraum verbleiben. Da alle zur Funktion notwendigen Komponenten vorhanden sind, ist eine Verbindung nach außen nicht erforderlich.

Es besteht aber nach einer anderen Ausführungsform der Erfindung auch die Möglichkeit, daß der Wirkstoff-Vorratsbehälter, vorzugsweise zusammen mit der Dosiereinrichtung und der Dosiersteuerung, von dem Sensor-Aktuatorkopf abgesetzt angeordnet ist und daß eine oder mehrere Verbindungsleitungen zwischen diesen Funktionsgruppen zum dosierten Zuführen des Wirkstoffes zu dem Sensor-Aktuatorkopf und zum Verbinden mit den Elektroden vorgesehen ist.
Der Sensor-Aktuatorkopf selbst kann bei dieser Ausführungsform besonders klein ausgebildet sein, so daß er auch an schwierig zugänglichen Stellen innerhalb des Körpers einsetzbar ist. Außerdem besteht hierbei die Möglichkeit, daß die zum Sensor-Aktuatorkopf abgesetzte Versorgungseinheit gut zugänglich angeordnet wird, so daß ein Nachfüllen mit medizinischem Wirkstoff, eine eventuell externe Stromzuführung und dergleichen, einfach realisierbar sind.

Vorzugsweise sind bei der Auflage- und Kontaktfläche des Sensor-Aktuatorkopfes, insbesondere randseitig, Haftregionen zum vorzugsweise adhäsiven Anhaften des Sensor-Aktuatorkopfes an dem zu behandelnden Gewebebereich vorgesehen.
Dadurch kann der Sensor-Aktuatorkopf durch einfaches Ansetzen und Andrücken an dem zu behandelnden Bereich befestigt werden und zusätzliche Befestigungsmaßnahmen sind dadurch entbehrlich.
Vorteilhaft ist es dabei, wenn die Haftregionen des Sensor-Aktuatorskopfes elektrisch leitend ausgebildet sind und gleichzeitig als Elektroden für die Iontophorese dienen.
Dies ist platzsparend und vereinfacht den Aufbau des Sensor-Aktuatorkopfes.
Zusätzliche Ausgestaltungen der Erfindung sind in den weiteren Unteransprüchen aufgeführt.
Nachstehend ist die Erfindung mit ihren wesentlichen Einzelheiten anhand der Zeichnungen noch näher erläutert.
Es zeigt:
- Figur 1: eine etwas schematisierte Darstellung einer erfindungsgemäßen Vorrichtung mit einem Sensor-Aktuatorkopf sowie einer dazu abgesetzt angeordneten und über eine Verbindungsleitung verbundenen Versorgungseinheit,
- Figur 2: eine Unterseitenansicht des in Figur 1 gezeigten Sensor-Aktuatorkopfes,
- Figur 3: eine Schnittdarstellung der Verbindungsleitung zwischen Sensor-Aktuatorkopf und Versorgungseinheit gemäß Figur 1,
- Figur 4: eine andere Ausführungsform der erfindungsgemäßen Vorrichtung in perspektivischer Darstellung,
- Figur 5: eine Unterseitenansicht der in Figur 4 gezeigten Vorrichtung, und
- Figur 6: eine röhrenförmige Ausbildung eines Sensor-Aktuatorkopfes mit Blick auf die Kontaktfläche für den Einsatz in Hohlorganen.

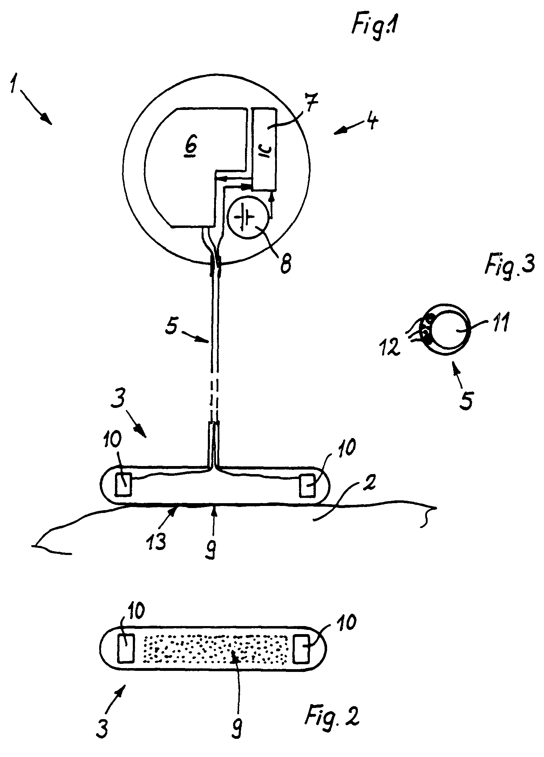

Eine in Figur 1 gezeigte Vorrichtung 1 dient zum Applizieren von medizinischem Wirkstoff im Bereich von lebendem Zellgewebe. Es kann sich dabei insbesondere um einen zu behandelnden Tumor 2 handeln, der in Figur 1 angedeutet ist.
Die Vorrichtung 1 weist einen Sensor-Aktuatorkopf 3, eine im Ausführungsbeispiel gemäß Figur 1 zu diesem Sensor-Aktuatorkopf 3 abgesetzte Versorgungseinheit 4 sowie eine Verbindungsleitung 5 zwischen Sensor-Aktuatorkopf 3 und Versorgungseinheit 4 auf. Innerhalb der Versorgungseinheit 4 befindet sich ein Wirkstoff-Vorratsbehälter 6 vorzugsweise mit einer hier nicht näher dargestellten Dosiereinrichtung sowie einer Dosiersteuerung 7 und eine Stromversorgung 8.
Der Sensor-Aktuatorkopf 3 weist an seiner Auflage- und Kontaktfläche 13 eine poröse Membrane 9 (vgl. auch Figur 2), eine Wirkstoff-Zuführung zu dieser Membrane 9 sowie benachbart zu der Membrane Sensoren 10 auf. Die Membrane 9 sowie die Sensoren 10 kontaktieren in Applikationsstellung den Behandlungsbereich. Innerhalb der Verbindungsleitung 5, die im praktischen Ausführungsbeispiel durch einen Katheterschlauch gebildet sein kann, kann der medizinische Wirkstoff vom Vorratsbehälter 6 der porösen Membrane 9 zugeführt werden und außerdem sind in der Verbindungsleitung 5 auch elektrische Verbindungen zwischen dem oder den Sensoren 10 und der Dosiersteuerung 7 untergebracht. Dies ist in der Querschnittdarstellung gemäß Figur 3 gut erkennbar. Der Verbindungsschlauch für den Wirkstoff ist hierbei mit 11 und die elektrischen Leitungen sind mit 12 bezeichnet.

Mit Hilfe der erfindungsgemäßen Vorrichtung kann medizinischer Wirkstoff direkt bei dem zu behandelnden Bereich, beispielsweise einem Tumor 2 appliziert werden. Dazu wird der Sensor-Aktuatorkopf 3 direkt auf den zu behandelnden Bereich aufgesetzt und über die poröse Membrane 9 kann dann in diesem Bereich der medizinische Wirkstoff zugeführt werden. Mit Hilfe der Sensoren 10 kann eine Kontrolle des Behandlungsbereiches erfolgen und aufgrund der Meßergebnisse kann über die mit den Sensoren 10 verbundene Dosiersteuerung 7 eine exakte Anpassung der Wirkstoffdosierung vorgenommen werden.
Zumindest einer der Sensoren 10 ist dabei ein pH-Sensor, da für eine erfolgreiche Immuntherapie beispielsweise die Überwachung des PH-Wertes der Mikroumgebung des zu behandelnden Bereiches und auch eine Beeinflussung dieser Umgebung durch Variation des pH-Wertes, insbesondere durch entsprechende Wirkstoffzugabe, von wesentlicher Bedeutung ist. Auch andere chemotherapeutische Konzepte sind darauf angewiesen, daß die steilen, extrazellulären pH-Gradienten abgebaut werden.
Es können auch innerhalb des Applikationsbereiches mehrere pH-Sensoren vorgesehen sein, wobei zwei oder mehrere pH-Sensoren für eine extrazelluläre Gradientenmessung zueinander beabstandet angeordnet sein können.

Als pH-Sensor werden vorzugsweise ionenselektive Feldeffekttransistoren (IS-FET) eingesetzt.
Außerdem können noch weitere Sensoren 10, insbesondere Ionen- oder Molekularsensoren vorgesehen sein, um noch aussagekräftigere Meßergebnisse der Mikroumgebung des zu behandelnden Bereiches zu erhalten. Damit ist dann eine sehr gezielte, wirksame Behandlung möglich.

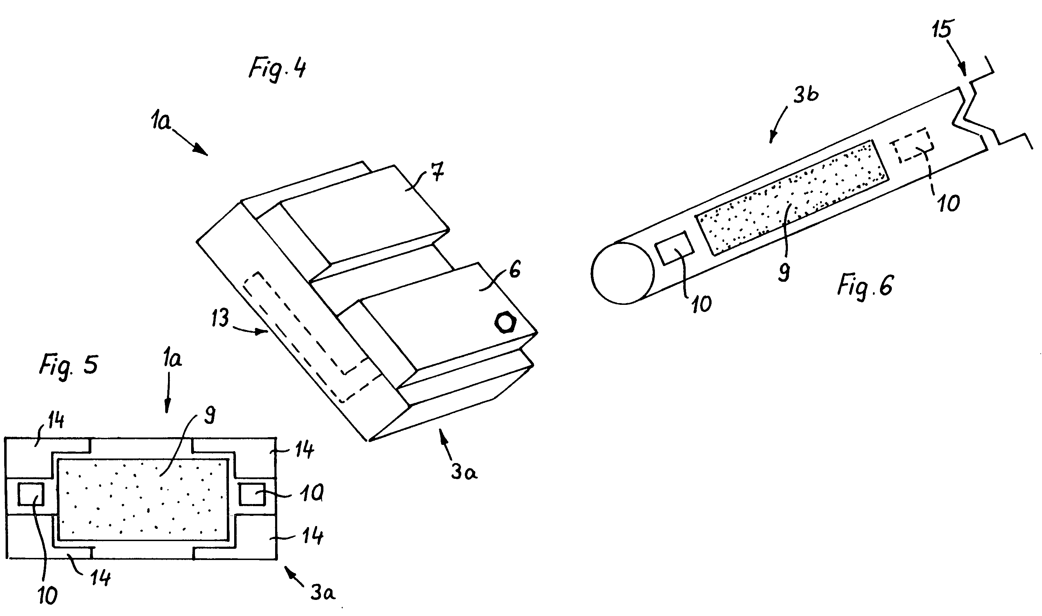

Die Figuren 4 und 5 zeigen eine abgewandelte Ausführungsform einer Vorrichtung 1a, bei der der Sensor-Aktuatorkopf 3a eine komplette Funktionseinheit bildet. Dieser Sensor-Aktuatorkopf 3a beinhaltet auch alle Baugruppen, die bei dem Ausführungsbeispiel gemäß Figur 1 in der Versorgungseinheit 4 untergebracht sind.
Bei dieser Ausführungsform der Vorrichtung 1a ergibt sich eine kompakte Einheit, die innerhalb des Körpers über einen entsprechenden Behandlungszeitraum als autarke Einheit verbleiben kann.
Die Unterseitenansicht gemäß Figur 5 zeigt noch im Bereich der Auflage- und Kontaktfläche 13, innerhalb der sich auch die poröse Membrane 9 sowie der oder die Sensoren 10 befinden, in den Eckbereichen beziehungsweise randseitig angeordnete Haftregionen 14. Mittels dieser Haftregionen 14 kann der Sensor-Aktuatorkopf 3a beziehungsweise auch der Sensor-Aktuatorkopf 3 gemäß Figur 1 und 2 an den zu behandelnden Bereich angedrückt werden und wird dann durch adhäsives Anhaften gehalten.
Die Haftregionen 14 können auch für iontophoretische Zwecke verwendet werden und sind dann elektrisch leitend ausgebildet und über Anschlußleitungen mit einer Spannungsquelle verbunden. Als Stromversorgung für die Dosiersteuerung und dergleichen kann eine auswechselbare oder aufladbare Batterie 8 (Figur 1) vorgesehen sein oder aber es besteht auch die Möglichkeit, daß eine thermoelektrische oder eine unter Zuhilfenahme der Körperflüssigkeit gebildete, galvanische Stromversorgung vorgesehen ist. Thermoelektrische oder galvanische Stromversorgungen sind insbesondere in Verbindung mit der in Figur 4 und 5 gezeigten Ausführungsform der erfindungsgemäßen Vorrichtung vorteilhaft.

Figur 6 zeigt noch eine weitere Ausführungsvariante eines Sensor-Aktuatorkopfes 3b, der im wesentlichen röhrenförmig ausgebildet ist und an seiner äußeren Mantelfläche eine streifenförmig längsorientierte Auflage- und Kontaktfläche aufweist. Diese Ausführungsform kommt insbesondere zur Behandlung innerhalb von Hohlorganen zur Anwendung. Erwähnt sei hierbei, daß auch mehrere Auflage- und Kontaktflächen am Umfang des röhrenförmigen Sensor-Aktuatorkopfes verteilt vorgesehen sein können. Auch die anderen Ausführungsformen von Applikationsköpfen können mit mehreren Auflage- und Kontaktflächen und innerhalb von diesen befindlichen porösen Membranen 9 und Sensoren 10 ausgerüstet sein.
An einem Ende des röhrenförmigen Sensor-Aktuatorkopfes 3b ist noch ein Anschluß 15 für einen externen Wirkstoffbehälter, insbesondere zum Nachfüllen von Wirkstoff angedeutet.

## Patentansprüche

1. Vorrichtung zum Behandeln von lebendem Zellgewebe, wobei die Vorrichtung einen Sensor-Aktuatorkopf (3,3a,3b) mit einem pH-Sensor und einer Wirkstoffabgabeeinrichtung mit einem einen Wirkstoff bevorratenden Behälter (6) aufweist, und wobei der pH-Sensor und die Wirkstoffabgabeeinrichtung zur Wirkstoffdosierung in Abhängigkeit der pH-Wertmessung an eine Steuereinrichtung angeschlossen sind, **dadurch gekennzeichnet, daß** der Sensor-Aktuatorkopf (3,3a,3b) zumindest mit dem pH-Sensor (10) und der Wirkstoffabgabeeinrichtung derart ausgebildet ist, daß er innerhalb des Körpers im tumorösen Gewebebereich einsetzbar ist und diesen Behandlungsbereich in Applikationsstellung mit einer Auflage- und Kontaktfläche (13) direkt kontaktiert, daß der pH-Sensor (10) zur Bestimmung der Ansäuerung der unmittelbaren Umgebung dieses tumorösen Gewebebereichs angeordnet ist, und daß der Wirkstoff ein solcher zur Variation des pH-Wertes der unmittelbaren Umgebung des tumorösen Gewebebereichs und/oder zur Reduzierung der Ansäuerung der Tumorzellen selbst ist.

2. Vorrichtung insbesondere nach Anspruch 1, **dadurch gekennzeichnet, daß** am Sensor-Aktuatorkopf Elektroden zur physikalischen Beeinflussung des tumorösen Gewebebereiches durch elektrische und/oder elektromagnetische Felder mittels Iontophorese zur Anhebung des gemessenen pH-Wertes in der unmittelbaren Umgebung eines tumorösen Gewebebereiches vorgesehen sind.

3. Vorrichtung nach Anspruch 1, **dadurch gekennzeichnet, daß** die Wirkstoffabgabeeinrichtung vorzugsweise wenigstens eine die Auflage- und Kontaktfläche bildende poröse Membrane (9) und eine Wirkstoff-Zuführung zu dieser Membrane aufweist und daß sich bei der Wirkstoff-Zuführung eine Dosiereinrichtung befindet, die an eine Dosiersteuerung (7) angeschlossen ist.

4. Vorrichtung nach Anspruch 3, **dadurch gekennzeichnet, daß** der oder die Sensoren (10) benachbart zu der Membrane (9) angeordnet sind.

5. Vorrichtung nach einem der Ansprüche 1 bis 4, **dadurch gekennzeichnet, daß** bei der Auflage- und Kontaktfläche (13) für den zu behandelnden Gewebebereich wenigstens zwei Elektroden zu iontophoretischen Zwecken vorgesehen sind, die über elektrische Leitungen mit einer Spannungsquelle verbunden sind.

6. Vorrichtung nach einem der Ansprüche 1 bis 5, **dadurch gekennzeichnet, daß** bei der Auflage- und Kontaktfläche (13) des Sensor-Aktuatorkopfes (3,3a,3b), insbesondere randseitig, Haftregionen (14) zum vorzugsweise adhäsiven Anhaften des Sensor-Aktuartorkopfes an dem zu behandelnden Gewebebereich (2) vorgesehen sind und daß die Haftregionen (14) des Sensor-Aktuatorkopfes gegebenenfalls elektrisch leitend ausgebildet sind und gleichzeitig als Elektrode für die Iontophorese dienen.

7. Vorrichtung nach einem der Ansprüche 1 bis 6, **dadurch gekennzeichnet, daß** der pH-Sensor auf Halbleiterbasis oder auf der Basis einer Leitfähigkeits- und Impedanzmessung ausgebildet ist und daß bei einem ph-Sensor auf Halbleiterbasis für diesen vorzugsweise wenigstens ein ionenselektiver Feldeffekttransistor vorgesehen ist.

8. Vorrichtung nach einem der Ansprüche 1 bis 7, **dadurch gekennzeichnet, daß** zusätzlich zu wenigstens einem pH-Sensor wenigstens ein weiterer Sensor, insbesondere ein Ionen- oder Molekularsensor vorgesehen ist.

9. Vorrichtung nach einem der Ansprüche 3 bis 8, **dadurch gekennzeichnet, daß** der Sensor-Aktuatorkopf (1a) eine komplette Funktionseinheit bildet und daß diese insbesondere wenigstens einen Wirkstoff-Vorratsbehälter (6), eine oder mehrere, mit der porösen Membrane (9) oder dergleichen verbundene Dosiereinrichtung(en) mit Dosiersteuerung (7) sowie zumindest einen pH-Sensor (10) aufweist.

10. Vorrichtung nach Anspruch 8, **dadurch gekennzeichnet, daß** der als Funktionseinheit ausgebildete Sensor-Aktuatorkopf wenigstens einen pH-Sensor (10), wenigstens zwei Elektroden zu iontophoretischen Zwecken, eine Spannungsquelle sowie eine Steuereinrichtung aufweist.

11. Vorrichtung nach einem der Ansprüche 1 bis 10, **dadurch gekennzeichnet, daß** der Sensor-Aktuatorkopf (3b) im wesentlichen röhrenförmig ausgebildet ist und insbesondere an seiner Mantelfläche zumindest eine vorzugsweise streifenförmig längsorientierte Auflage- und Kontaktfläche (13) für den zu behandelnden Gewebebereich aufweist.

12. Vorrichtung nach einem der Ansprüche 1 bis 8 oder 11, **dadurch gekennzeichnet, daß** der Wirkstoff-Vorratsbehälter (6), vorzugsweise zusammen mit der Dosiereinrichtung und der Dosiersteuerung (7), von dem Sensor-Aktuatorkopf (3) abgesetzt angeordnet ist und daß eine oder mehrere Verbindungsleitungen (5) zwischen diesen Funktionsgruppen zum insbesondere dosierten Zuführen des Wirkstoffes zu dem Sensor-Aktuatorkopf (3) und/oder zum Verbinden mit den Elektroden vorgesehen ist.

13. Vorrichtung nach einem der Ansprüche 1 bis 12, **dadurch gekennzeichnet, daß** an dem Sensor-Aktuatorkopf (3b) ein Anschluss (15) für einen externen Wirkstoffbehälter, insbesondere zum Nachfüllen von Wirkstoff vorgesehen ist.

14. Vorrichtung nach einem der Ansprüche 1 bis 13, **dadurch gekennzeichnet, daß** als Stromversorgung für die Vorrichtung eine im Sensor-Aktuatorkopf oder in einer von diesem abgesetzten Funktionseinheit befindliche Batterie und/oder eine thermoelektrische und/oder eine unter Zuhilfenahme der Körperflüssigkeit gebildete, galvanische Stromversorgung vorgesehen ist.

## Claims

1. A device for treating living cell tissue, where the device comprises a sensor actuator head (3, 3a, 3b) having a pH sensor and a drug dispensing device having a reservoir (6) storing the drug, and where the pH sensor and the drug dispensing device are connected to a control device for dosing the drug as a function of the pH value measurement,
**characterised in that** the sensor actuator head (3, 3a, 3b) at least with the pH sensor (10) and the drug dispensing device is constructed in such a manner that it can be inserted inside the body in the tumorous tissue region and in the application position in this contacts this treatment area with a supporting and contact surface (13),
**in that** the pH sensor (10) is provided to determine the acidulation of the direct vicinity of this tumorous tissue region,
and **in that** the drug is one intended for the variation of the pH value in the direct vicinity of the tumorous tissue region and/or for the reduction of the acidulation of the tumour cells.

2. A device especially according to Claim 1,
**characterised in that** at the sensor actuator head electrodes are provided for physically influencing the tumorous tissue region by electrical and/or electromagnetic fields by means of iontophoresis to raise the measured pH value in the direct vicinity of a tumorous tissue region.

3. A device according to Claim 1,
**characterised in that** the drug dispensing device preferably comprises at least one porous membrane (9) forming the supporting and contact surface and a drug supply to this membrane
**and in that** during the supply of drug a dosing device is provided, which is connected to a dosage control device (7).

4. A device according to Claim 3,
**characterised in that** the sensor or sensors (10) are disposed adjacent to the membrane (9).

5. A device according to one of Claims 1 to 4,
**characterised in that** for the supporting and contract surface (13) for the tissue area to be treated, at least two electrodes, which are connected via electric lines to a voltage source, are provided for iontophoretic purposes.

6. A device according to one of Claims 1 to 5,
**characterised in that** for the supporting and contact surface (13) of the sensor actuator head (3, 3a, 3b), adhesive regions (14) are provided, in particular at the edge, for the preferably adhesive bonding of the sensor actuator head to the tissue area (2) to be treated
**and in that** the adhesive regions (14) of the sensor actuator head possibly have an electrically conductive construction and at the same time serve as an electrode for the iontophoresis.

7. A device according to one of Claims 1 to 6,
**characterised in that** the pH sensor is constructed on a semiconductor base or on the base/basis of a conductivity and impedance measurement
**and in that** in the case of a pH sensor on a semiconductor base at least one ion-selective field-effect transistor is preferably provided for this.

8. A device according to one of Claims 1 to 7,
**characterised in that** in addition to at least one pH sensor, at least one further sensor is provided, in particular an ion or molecular sensor.

9. A device according to one of Claims 3 to 8,
**characterised in that** the sensor actuator head (la) forms a complete functional unit
**and in that** this comprises in particular at least one reservoir (6) for the drug, one or more dosing device(s) with dosage control device (7) connected to the porous membrane (9) or the like and also at least one pH sensor (10).

10. A device according to Claim 8,
**characterised in that** the sensor actuator head constructed as a functional unit comprises at least one pH sensor(10), at least two electrodes for iontophoretic purposes, a voltage source and also a control device.

11. A device according to one of Claims 1 to 10,
**characterised in that** the sensor actuator head (3b) has a substantially tubular construction and, in particular on its shell surface, comprises at least one supporting and contact surface (13) which is longitudinally orientated like a strip for the tissue area to be treated.

12. A device according to one of Claims 1 to 8 or 11,
**characterised in that** the reservoir (6) for the drug, preferably together with the dosing device and the dosage control device (7), is disposed removed from the sensor actuator head (3)
**and in that** one or more connecting lines (5) is provided between these functional groups, in particular to supply the drug in doses to the sensor actuator head (3) and/or for connection with the electrodes.

13. A device according to one of Claims 1 to 12,
**characterised in that** a connection (15) for an external drug reservoir, in particularly for topping up the drug, is provided at the sensor actuator head (3b).

14. A device according to one of Claims 1 to 13,
**characterised in that** a battery situated in the sensor actuator head or in a functional unit removed therefrom and/or a thermoelectric and/or a galvanic current supply, which is formed with the aid of body fluid, is provided as the current supply for the device.

## Revendications

1. Dispositif de traitement du tissu cellulaire vivant, dans lequel le dispositif présente une tête de commande à capteur (3, 3a, 3b) avec un capteur de pH et un dispositif d'administration de substance active comportant un récipient de réserve de substance active (6), et dans lequel le capteur de pH et le dispositif d'administration de substance active sont connectés, en vue du dosage de la substance active en fonction de la mesure du pH, à un dispositif de réglage,
**caractérisé en ce que**
la tête de commande à capteur (3, 3a, 3b) est formée au moins avec le capteur de pH (10) et le dispositif d'administration de substance active, de manière qu'elle soit utilisable à l'intérieur du corps dans la zone tissulaire tumorale et que cette zone de traitement soit en contact direct dans la position d'application avec une surface de dépôt et de contact (13), le capteur de pH (10) pour la détermination de l'acidification est disposé dans l'environnement immédiat de cette zone tissulaire tumorale, et la substance active est appropriée à la variation du pH de l'environnement immédiat de la zone tissulaire tumorale et/ou à la réduction de l'acidification des cellules tumorales.

2. Dispositif en particulier selon la revendication 1,
**caractérisé en ce que**
sur la tête de commande du capteur sont prévues des électrodes permettant d'influer physiquement sur la zone tissulaire tumorale par des champs électriques et/ou électromagnétiques au moyen d'une ionophorèse pour élever la valeur du pH mesuré dans l'environnement immédiat d'une zone tissulaire tumorale.

3. Dispositif selon la revendication 1,
**caractérisé en ce que**
le dispositif d'administration de substance active présente de préférence au moins une membrane poreuse (9) formant la surface de dépôt et de contact et une amenée de substance active à cette membrane, et
sur l'amenée de substance active se trouve un dispositif de dosage qui est connecté à une commande de dosage (7).

4. Dispositif selon la revendication 3,
**caractérisé en ce que**
le ou les capteurs (10) sont disposés au voisinage de la membrane (9).

5. Dispositif selon l'une des revendications 1 à 4,
**caractérisé en ce que**
sur la surface de dépôt et de contact (13) pour la zone tissulaire à traiter sont prévues au moins deux électrodes dans des buts ionophorétiques, qui sont connectées par l'intermédiaire de lignes électriques avec une source de tension.

6. Dispositif selon l'une des revendications 1 à 5,
**caractérisé en ce que**
sont prévues sur la surface de dépôt et de contact (13) de la tête de commande à capteur (3, 3a, 3b), en particulier sur les bords, des régions d'adhérence (14) pour faire adhérer de préférence par collage la tête de commande à capteur sur la zone tissulaire à traiter (2), et
les régions d'adhérence (14) de la tête d'adhérence à capteur sont le cas échéant formées de manière à être électriquement conductrices et à servir simultanément d'électrode pour l'ionophorèse.

7. Dispositif selon l'une des revendications 1 à 6,
**caractérisé en ce que**
le capteur de pH est formé à base de semi-conducteur ou à base d'une mesure de conductivité et d'impédance, et
sur un capteur de pH à base de semi-conducteur est prévu pour celui-ci de préférence au moins un transistor à effet de champ à sélectivité ionique.

8. Dispositif selon l'une des revendications 1 à 7,
**caractérisé en ce qu'**
outre le capteur de pH, présent en au moins un exemplaire, est au moins prévu un autre capteur, en particulier un capteur ionique ou moléculaire.

9. Dispositif selon l'une des revendications 3 à 8,
**caractérisé en ce que**
la tête de commande à capteur (1a) forme une unité fonctionnelle complète, et
celle-ci comprend en particulier au moins un récipient de réserve de substance active (6), un ou plusieurs dispositifs liés à la membrane poreuse (9) ou analogue avec un réglage du dosage (7) ainsi qu'au moins un capteur de pH (10).

10. Dispositif selon la revendication 8,
**caractérisé en ce que**
la tête de commande à capteur formée en unité fonctionnelle présente au moins un capteur de pH (10), au moins deux électrodes dans des buts ionophorétiques, une source de tension ainsi qu'un dispositif de réglage.

11. Dispositif selon l'une des revendications 1 à 10,
**caractérisé en ce que**
la tète de commande à capteur (3b) est formée de manière essentiellement tubulaire et présente en particulier sur sa surface d'enveloppe une surface de dépôt ou de contact (13) orientée de préférence de manière longitudinale en forme de ruban pour la zone tissulaire à traiter.

12. Dispositif selon l'une des revendications 1 à 8 ou 11,
**caractérisé en ce que**
le récipient de réserve de substance active (6), est disposé de préférence séparément de la tête de commande à capteur (3) et
une ou plusieurs lignes de connexion (5) sont prévues entre ces groupes fonctionnels en particulier pour amener de façon dosée la substance active à la tête de commandes à capteurs (3) et/ou aux fins de connexion avec les électrodes.

13. Dispositif selon l'une des revendications 1 à 12,
**caractérisé en ce que**
sur la tête de commande à capteur (3b) est prévu un raccord (15) pour un récipient de substance active externe, en particulier pour la recharge de substance active.

14. Dispositif selon l'une des revendications 1 à 13,
**caractérisé en ce que**
comme alimentation en courant pour le dispositif on prévoit une pile dans la tête d'alimentation à capteur ou dans une unité fonctionnelle qui en est séparée, et/ou une alimentation en courant galvanique thermoélectrique et/ou formée à l'aide, du liquide corporel.
